# EUROPEAN PATENT SPECIFICATION

(11) **EP 4 129 245 B1**
(45) Date of publication and mention of the grant of the patent: **04.11.2026**
(21) Application number: 22184425.1
(22) Date of filing: 12.07.2022
(51) Int. Cl.: A61F 2/95, A61F 2/966

(54) **MEDICAL DEVICE FOR THE DELIVERY OF MEDICAL DEVICES**
MEDIZINISCHE VORRICHTUNG ZUR EINFÜHRUNG VON MEDIZINISCHEN VORRICHTUNGEN
DISPOSITIF MÉDICAL D'INSERTION DE DISPOSITIFS MÉDICAUX

(30) Priority: 05.08.2021 US 202117444502
(43) Date of publication of application: 08.02.2023
(73) Proprietor: Covidien LP, Mansfield, MA 02048 (US)
(72) Inventor: Deen, Daniel, Irvine, CA 92617 (US); Nageswaran, Ashok, Irvine, CA 92617 (US)
(74) Representative: Gray, James

(56) References cited:
- US-A1- 2015 066 130
- US-A1- 2019 374 358

## Description

### TECHNICAL FIELD

The present technology relates to medical device delivery devices, systems, and methods.

### BACKGROUND

Walls of the vasculature, particularly arterial walls, may develop areas of pathological dilatation called aneurysms that often have thin, weak walls that are prone to rupturing. Aneurysms are generally caused by weakening of the vessel wall due to disease, injury, or a congenital abnormality. Aneurysms occur in different parts of the body, and the most common are abdominal aortic aneurysms and cerebral (e.g., brain) aneurysms in the neurovasculature. When the weakened wall of an aneurysm ruptures, it can result in death, especially if it is a cerebral aneurysm that ruptures.

Aneurysms are generally treated by excluding or at least partially isolating the weakened part of the vessel from the arterial circulation. For example, conventional aneurysm treatments include: (i) surgical clipping, where a metal clip is secured around the base of the aneurysm; (ii) packing the aneurysm with small, flexible wire coils (micro-coils); (iii) using embolic materials to "fill" an aneurysm; (iv) using detachable balloons or coils to occlude the parent vessel that supplies the aneurysm; and (v) intravascular stenting.

Intravascular stents are well known in the medical arts for the treatment of vascular stenoses or aneurysms. Stents are prostheses that expand radially or otherwise within a vessel or lumen to support the vessel from collapsing. Methods for delivering these intravascular stents are also well known.

Conventional methods of introducing a compressed stent into a vessel and positioning it within an area of stenosis or an aneurysm include percutaneously advancing a distal portion of a guiding catheter through the vascular system of a patient until the distal portion is proximate the stenosis or aneurysm. A second, inner catheter is advanced through the distal region of the guiding catheter. A stent delivery system is then advanced out of the distal region of the guiding catheter into the vessel until the distal portion of the delivery system carrying the compressed stent is positioned at the point of the lesion within the vessel. The compressed stent is then released and expanded so that it supports the vessel at the point of the lesion.

US2015/066130 A1 describes a stent delivery system that can include a core member, an introducer sheath, and a microcatheter. The core member can have a first section and a second section distal to the first section. The second section can have a bending stiffness per unit length that is less than a bending stiffness per unit length of the first section.

US2019/374358 A1 describes a stent delivery system that can include a core assembly sized for insertion into a corporeal lumen and configured for advancing a stent toward a treatment location in the corporeal lumen. The core assembly can include a longitudinally extending tube having a lumen and a helical cut extending along the tube. An elongate wire can extend through the tube lumen.

### SUMMARY

Conventional stent delivery systems can include a core member that assists with deploying a stent at a treatment site. The core member can be a multi-component assembly that includes a proximal portion in the form of a wire that can be gripped by a clinician, a middle portion in the form of a hypotube with flexibility enhancing cuts configured to extend through the patient's vasculature, and a distal portion in the form of a wire distally extending from a distal end of the hypotube. The stent can be positioned over the distal wire, where the stent can engage with the core member via one or more stent engagement members that are coupled to the distal wire. To ensure the stent is reliably and accurately deployed at the treatment site, the distal wire must be properly connected within the core member. The distal wire is typically connected within the core member by being soldered or welded to the hypotube. **In** these conventional stent delivery systems, the connection between the wire and the hypotube can fail. **In** severe cases, the distal wire can become disconnected from the hypotube and be left untethered from the rest of the delivery system while inside a blood vessel. These and other connection failures have been linked to the presence of flux within an inner diameter of the hypotube.

The present technology relates to medical delivery devices, systems, and methods configured to address the above-noted limitations of existing stent delivery systems as well as other issues. Some embodiments of the present technology, for example, are directed to a medical device delivery system comprising a joining element that can provide a stronger and more reliable connection between the wire and the hypotube within the core member. The joining element can be welded to an end of the hypotube and can interlock with the wire. This arrangement moves the solder location of the distal wire away from the interior of the hypotube and provides an interference fit for the distal wire instead of a soldered joint. As a result of this arrangement, the hypotube and distal wire within a core member are less susceptible to connection failures.

Additional features and advantages of the present technology are described below, and in part will be apparent from the description, or may be learned by practice of the present technology. The advantages of the present technology will be realized and attained by the structure particularly pointed out in the written description and claims hereof as well as the appended drawings. The subject technology is illustrated, for example, according to various aspects described below. These are provided as examples and do not limit the subject technology.

**In** one embodiment, a medical device delivery system is described. The medical device delivery system can include an elongated tubular member having a distal end portion, a proximal end portion, and a lumen extending therethrough and a joining element coupled to a distal end portion of the elongated tubular member. The joining element can include a bumper having a distal end portion configured to engage a proximal portion of a medical device and a proximal portion adjacent the distal end portion of the elongated tubular member, wherein the proximal portion defines a slot having a length along a first direction. The joining element can also include an aperture extending through the bumper, the aperture having a first cross-sectional dimension along a second direction and a second cross-sectional dimension along a third direction different from the second direction, the first cross-sectional dimension being greater than the second cross-sectional dimension, wherein the first direction is about orthogonal to the second direction. The medical device delivery system can also include an elongated shaft having a distal region and a flattened region proximal of the distal region, the flattened region having a greatest cross-sectional dimension that is smaller than the first cross-sectional dimension of the aperture but larger than the second cross-sectional dimension of the aperture, wherein the flattened region is at least partially received within the slot.

**In** one embodiment not of the present invention, a core assembly for a medical device delivery system is described. The core assembly can include an elongated tubular member having a distal end portion, a proximal end portion, and a lumen extending therethrough, and a joining element coupled to the distal end portion of the tubular member. The joining element can include: a bumper having a proximal face abutting the distal end portion of the elongated tubular member; a proximal portion extending proximal of the proximal face and into the lumen of the elongated tubular member; and an aperture extending through the bumper and the proximal portion, the aperture having a first cross-sectional dimension along a first radial axis and a second cross-sectional dimension along a second radial axis, the first cross-sectional dimension being greater than the second cross-sectional dimension. The core assembly can further include an elongated shaft coupled to the joining element. The elongated shaft can include: an intermediate portion extending distal to the bumper portion and configured to receive a medical device thereover; a proximal portion extending through the joining element aperture; and an engagement feature disposed along the proximal portion, the engagement feature having a first radially outermost dimension along a first radial direction and a second radially outermost dimension along a second radial direction, the first radially outermost dimension being smaller than the first cross-sectional dimension and the second-cross sectional dimension of the aperture, the second radially outermost dimension being smaller than the first cross-sectional dimension of the aperture but larger than the second cross-sectional dimension of the aperture.

**In** one embodiment not of the present invention, a medical device delivery system is described. The medical device delivery system can include a hypotube having a proximal portion, a distal portion, and a lumen extending therethrough and a joining element positioned at the distal portion of the hypotube. The joining element can include: a bumper portion having a proximal-facing surface abutting a distal end of the hypotube; a proximal portion extending proximally from the distal portion such that the proximal portion is positioned within the lumen of the hypotube; and an aperture extending through the bumper and proximal portions, wherein the aperture has a first cross-sectional dimension along a first radial direction and a second cross-sectional dimension along a second radial direction, the second cross-sectional dimension being smaller than the first cross-sectional dimension. The medical device delivery system can also include an elongated member having a proximally located attachment portion including a retention region extending through the aperture and a widened region extending laterally away from a longitudinal axis of the elongate member to a greater extent than the retention region, wherein the widened region is configured to fit through the aperture in a first orientation and to collide with the bumper and the proximal portions in a second orientation.

**In** one embodiment not of the present invention, a medical device delivery system is described. The medical device delivery system can include an elongated tubular member having a distal end portion, a proximal end portion, and a lumen extending therethrough, and a joining element coupled to a distal end portion of the elongated tubular member. The joining element can include: a bumper having a distal end portion configured to engage a proximal portion of a medical device and a proximal portion adjacent the distal end portion of the elongated tubular member; a nose having a body extending between a proximal end portion and a distal end portion, the proximal end portion of the nose being adjacent the distal end portion of the bumper; and an aperture extending through the bumper, the aperture having a first cross-sectional dimension along a first direction and a second cross-sectional dimension along a second direction different from the first direction, the first cross-sectional dimension being greater than the second cross-sectional dimension. The medical device delivery system can also include an elongated shaft having a distal region and a flattened region proximal of the distal region, the flattened region having a greatest cross-sectional dimension that is smaller than the first cross-sectional dimension but larger than the second cross-sectional dimension.

In one embodiment not of the present invention, a method of assembling a medical device delivery system is described. The method can include coupling a bumper to a distal end portion of an elongated tubular member. The bumper can include: a body having a proximal face abutting the distal end portion of the elongated tubular member and a distal face configured to abut a proximal portion of a medical device; a proximal portion extending proximal of the proximal face and into a lumen of the elongated tubular member, the proximal portion having a slot with a length along a first radial axis; and a aperture extending through the body and the proximal portion, the aperture having a first cross-sectional dimension along a second radial axis and a second cross-sectional dimension along a third radial axis, the first cross-sectional dimension being greater than the second cross-sectional dimension. The method can also include orienting an elongated shaft with the aperture. The elongated shaft can have an engagement feature disposed along a proximal portion of the elongated shaft, the engagement feature having a first radially outermost dimension along a first radial direction and a second radially outermost dimension along a second radial direction. Orienting the elongated shaft with the aperture can include aligning the second radial direction with the second radial axis. The method can also include moving the engagement feature of the elongated shaft distally through the first aperture; aligning the second radial direction with the first radial axis; and moving the engagement feature of the elongated shaft proximally until the engagement feature is received within the slot of the proximal portion of the bumper.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A is a schematic illustration of a medical device delivery system in accordance with one or more embodiments of the present technology.
FIG. 1B is a partial schematic view of the medical device delivery system of FIG. 1A within a blood vessel.
FIG. 2A is a schematic illustration of a bumper in accordance with one or more embodiments of the present technology.
FIG. 2B is an end view of the bumper of FIG. 2A.
FIG. 3A is a schematic illustration of an elongated shaft in accordance with one or more embodiments of the present technology.
FIG. 3B is an end view of the elongated shaft of FIG. 3A.
FIG. 4A is a schematic illustration of a nose in accordance with one or more embodiments of the present technology.
FIG. 4B is an end view of the nose of FIG. 4A.
FIG. 5A is a schematic illustration of an elongated shaft being inserted into a bumper in accordance with one or more embodiments of the present technology.

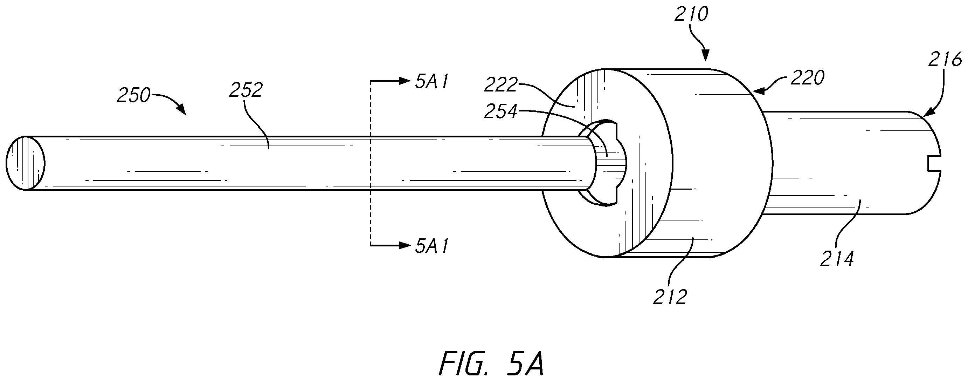

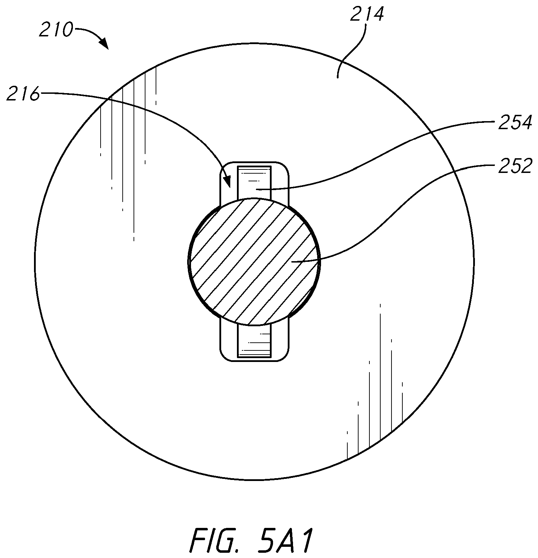

FIG. 5A1 is a cross-sectional view along the line SA1 from FIG. 5A.

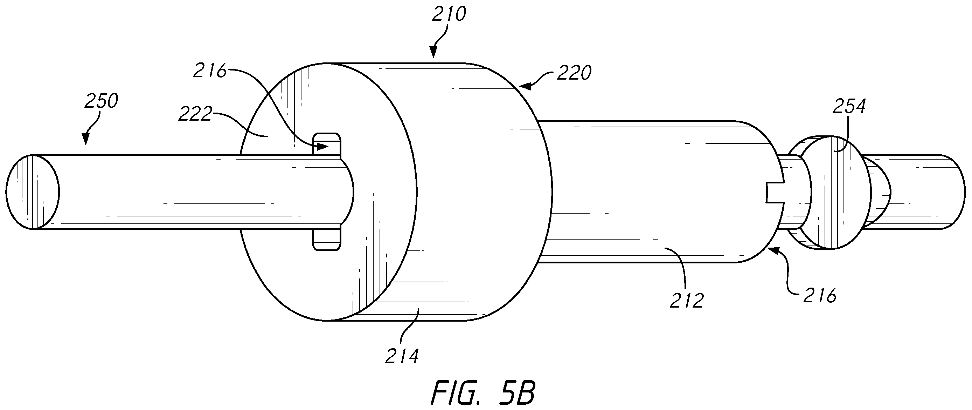

FIG. 5B is a schematic illustration of an elongated shaft inserted into a bumper in accordance with one or more embodiments of the present technology.

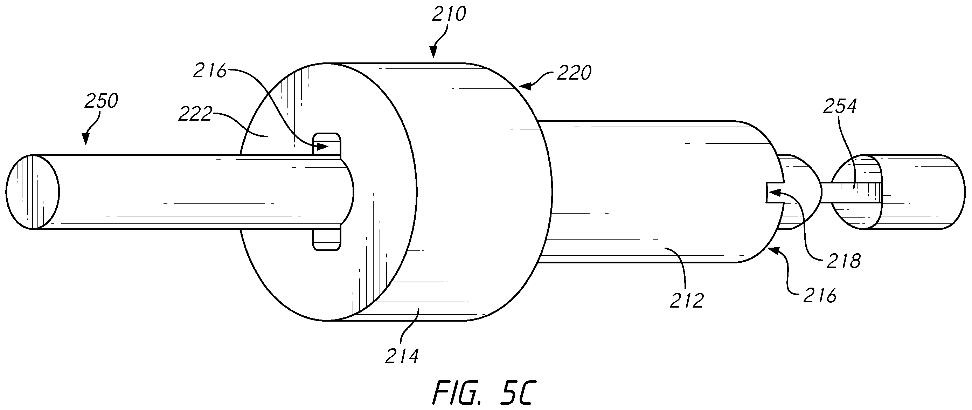

FIG. 5C is a schematic illustration of an elongated shaft being reoriented in accordance with one or more embodiments of the present technology.

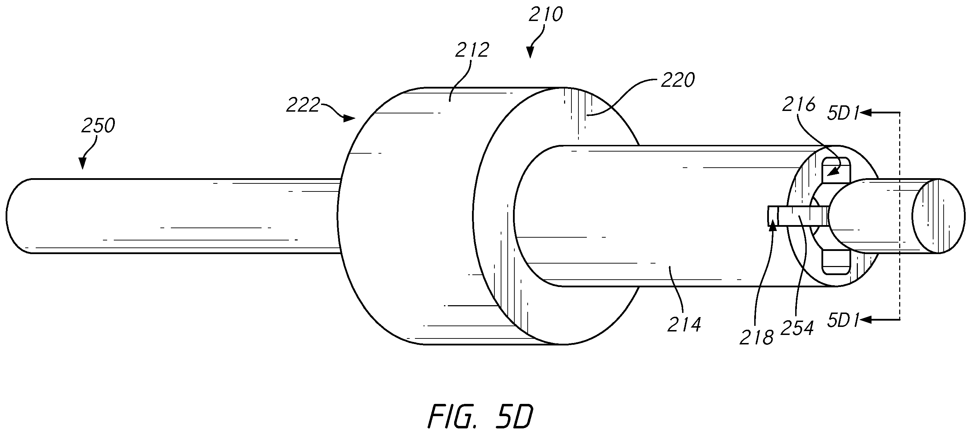

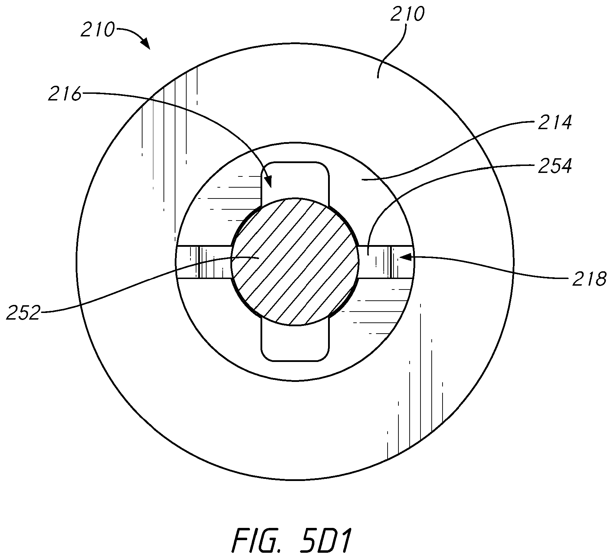

FIG. 5D is a schematic illustration of a widened region being received within a slot in accordance with one or more embodiments of the present technology.
FIG. 5D1 is a cross-sectional view along the line SD1 from FIG. 5D.

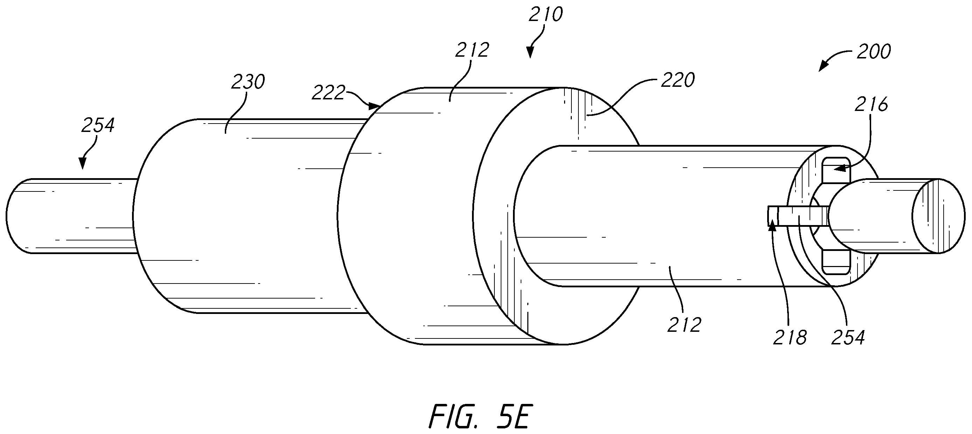

FIG. 5E is a schematic illustration of a nose being positioned over the elongated shaft in accordance with one or more embodiments of the present technology.

### DETAILED DESCRIPTION

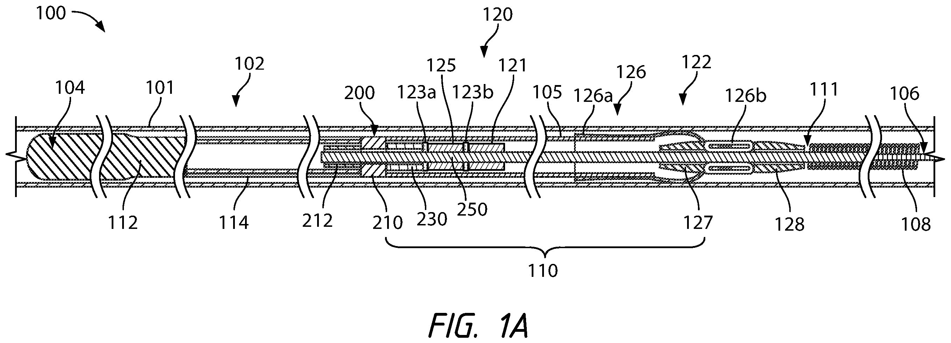

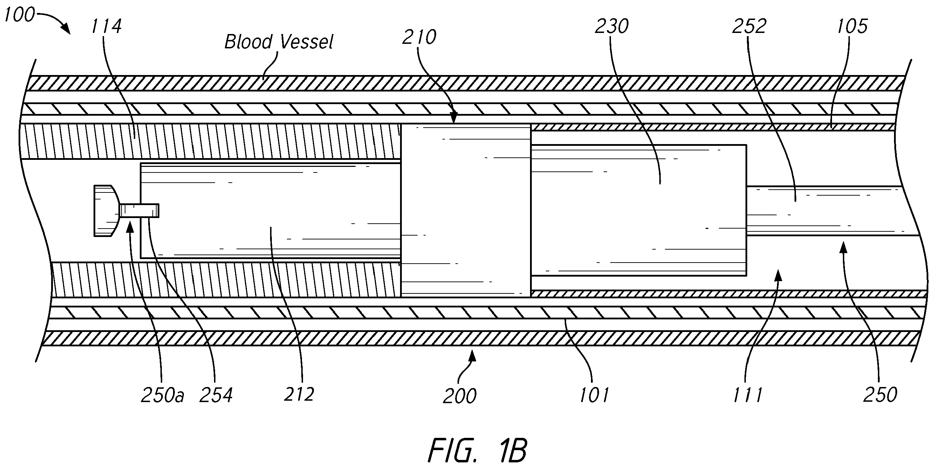

The present technology relates to medical delivery devices, systems, and methods configured to address the above-noted limitations of existing stent delivery systems as well as other issues. Details of one or more embodiments of the technology are described below with reference to FIGS. 1-5E. As used herein, the terms "distal" and "proximal" define a position or direction with respect to a clinician or a clinician's control device (e.g., a handle of a delivery catheter). For example, the terms, "distal" and "distally" refer to a position distant from or in a direction away from a clinician or a clinician's control device along the length of device. In a related example, the terms "proximal" and "proximally" refer to a position near or in a direction toward a clinician or a clinician's control device along the length of device.

FIG. 1A is a schematic illustration of a medical device delivery system 100 ("system 100") configured in accordance with one or more embodiments of the present technology. FIG. 1B is a partial schematic view of the medical device delivery system of FIG. 1A within a blood vessel. This system 100 may be used to deliver and/or deploy a medical device, such as but not limited to a stent, into a hollow anatomical structure, such as a blood vessel. The stent can comprise a braided stent or other form of stent such as a woven stent, knit stent, laser-cut stent, roll-up stent, etc. The stent can optionally be configured to act as a "flow diverter" device for treatment of aneurysms, such as those found in blood vessels including arteries in the brain or within the cranium, or in other locations in the body such as peripheral arteries. The stent can optionally be similar to any of the versions or sizes of the PIPELINE^{™} Embolization Device marketed by Medtronic Neurovascular of Irvine, California USA. The stent can alternatively comprise any suitable tubular medical device and/or other features, as described herein. In some embodiments, the stent can be any one of the stents described in U.S. Application No. 15/892,268, filed February 8, 2018, titled VASCULAR EXPANDABLE DEVICES.

With reference to FIGS. 1A and 1B, the system 100 can comprise a core member or core assembly 102 configured to extend generally longitudinally through the lumen 111 of an elongate catheter 101. The core member 102 can have a proximal region 104 and a distal region 106, which can optionally include a tip coil 108. The core member 102 can also comprise an intermediate portion 110 located between the proximal region 104 and the distal region 106. The intermediate portion 110 is the portion of the core member 102 onto or over which the stent 105 extends when the core member 102 is in the pre-deployment configuration as shown in FIG. 1A.

The delivery system 100 can include and/or be used with any number of catheters 101. For example, the catheter can optionally comprise any of the various lengths of the MARKSMAN^{™} catheter available from Medtronic Neurovascular of Irvine, California USA. The catheter can optionally comprise a microcatheter having an inner diameter of about 0.030 inches (0.762 mm) or less, and/or an outer diameter of 3 French (1 mm) or less near the distal region Instead of or in addition to these specifications, the catheter can comprise a microcatheter which is configured to access the internal carotid artery, or another location within the neurovasculature distal of the internal carotid artery.

The core member 102 can generally comprise any member(s) with sufficient flexibility and column strength to move the stent 105 or other medical device through a surrounding catheter 101. The core member 102 can therefore comprise a wire, tube (e.g., hypotube), braid, coil, or other suitable member(s), or a combination of wire(s), tube(s), braid(s), coil(s), etc. The embodiment of the core member 102 depicted in FIG. 1A is of multi-member construction, comprising a wire 112 with a tube 114 surrounding the wire 112 along at least a portion of its length. For example, a distal portion of the wire 112 can be positioned within the tube 114 so that the tube 114 surrounds the distal portion of the wire 112. An outer layer of lubricious material such as PTFE (polytetrafluoroethylene or TEFLON^{™}) or other lubricious polymers, can cover some or all of the tube 114 and/or wire 112. The wire 112 may taper or vary in diameter along some or all of its length. The wire 112 may include one or more fluorosafe markers (not shown), and such marker(s) can be located on a portion of the wire 112 that is not covered by the outer layer of lubricious material (e.g., proximal of the outer layer). This portion of the wire 112 marked by the marker(s), and/or proximal of any outer layer, can comprise a bare metal outer surface.

The core member 102 can comprise a stent coupling assembly 120 and/or a distal interface assembly 122 that can interconnect the stent 105 with the core member 102. In some embodiments, a joining element 200 couples the stent coupling assembly 120 and/or distal interface assembly 122 with the core member 102. As will be described in more detail, the joining element 200 can couple with the tube 114. For example, the joining element 200 can be welded to a distal end portion of the tube 114. The joining element 200 can be interlocked with an elongated shaft 250 that extends through the intermediate portion 110 and distal region 106 of the core member 102. The stent coupling assembly 120 can be coupled with the elongated shaft 250 within the intermediate portion 110 so that the stent coupling assembly 120 engages the stent 105. The distal interface assembly 122 can be coupled with the elongated shaft 250 distal to the stent 105 so that the distal interface assembly 122 engages with a distal portion of the stent 105.

The stent coupling assembly 120 can comprise one or more engagement members 123a, 123b (collectively "engagement members 123") that may underlie and mechanically engage or interlock with the stent 105. In this manner, the stent coupling assembly 120 cooperates with an overlying inner surface of a surrounding catheter 101 to engage the stent 105 such that the stent coupling assembly 120 can move the stent 105 along and within the catheter 101, e.g., as the user pushes the core member 102 distally and/or pulls the core member 102 proximally relative to the catheter 101, resulting in a corresponding distal and/or proximal movement of the stent 105 within the elongate shaft lumen 111.

The stent coupling assembly 120 can include one or more restraints 121 that are fixed to the core member 102 (e.g., to the elongated shaft 250 thereof in the depicted embodiment) so as to be immovable relative to the core member 102, either in a longitudinal/sliding manner or a radial/rotational manner. The stent coupling assembly 120 can also include a plurality of engagement members 123 separated by one or more spacers 125. For example, the stent coupling assembly 120 can include a first engagement member 123a and a second engagement member 123b separated by a spacer 125.

The engagement members 123 and/or the spacers 125 can be coupled to (e.g., mounted on) the core member 102 so that the stent coupling assembly 120 can rotate about the longitudinal axis of the core member 102 (e.g., of the intermediate portion 110), and/or move or slide longitudinally along the core member 102. For example, the engagement members 123 and the spacer 125 can be coupled to the elongated shaft 250 so that the engagement members 123 and the spacer 125 can rotate about the elongated shaft 250. In some embodiments, the stent 105 can be moved distally or proximally within an overlying catheter 101 via the stent coupling assembly 120. In some embodiments, the stent 105 can be resheathed via the stent coupling assembly 120 after partial deployment of the stent 105 from a distal opening of the catheter 101.

With continued reference to FIG. 1A, the distal interface assembly 122 can comprise a distal cover 126. In some examples, the distal cover 126 can take the form of, for example, a distal engagement member, a distal device cover, or distal stent cover. The distal cover 126 can be configured to reduce friction between the stent 105 (e.g., a distal portion thereof) and the inner surface of a surrounding catheter 101. For example, the distal cover 126 can be configured as a lubricious, flexible structure having a free first end or section 126a that can extend over at least a portion of the stent 105 and/or intermediate portion 110 of the core member 202, and a fixed second end or section 126b that can be coupled (directly or indirectly) to the core member 102. In some embodiments, the distal cover 126 is rotatably coupled to the core member 102.

The distal cover 126 can have a first (e.g., delivery) position, configuration, or orientation in which the distal cover 126 can extend proximally from the second section 126b or its (direct or indirect) attachment to the core member 102, and at least partially surround or cover a distal portion of the stent 105. The distal cover 226 can be movable from the first orientation to a second (e.g., resheathing) position, configuration, or orientation (not shown) in which the distal cover can be everted such that the first end 126a of the distal cover is positioned distally relative to the second end 126b of the distal cover 126 to enable the resheathing of the core member 102, either with the stent 105 carried thereby, or without the stent 105.

In some embodiments, the distal interface assembly can include a proximal and distal restraint 127, 128. One or both of the proximal and distal restraints 127, 128 can have an outside diameter or other radially outermost dimension that is smaller than the (e.g., pre-deployment) outside diameter or other radially outermost dimension of the distal cover 126, so that one or both of the restraints 127, 128 will tend not to bear against or contact the inner surface of the catheter 101 during operation of the core member 102. Alternatively, it can be preferable to make the outer diameters of the restraints 127 and 128 larger than the largest radial dimension of the pre-deployment distal cover 126, and/or make the outer diameter of the proximal restraint 127 larger than the outer diameter of the distal restraint 128. This configuration allows easy and smooth retrieval of the distal cover 126 and the restraints 127, 128 back into the elongate shaft post stent deployment.

The distal cover 126, the proximal restraint 127, and the distal restraint 128 can be coupled to (e.g., mounted on) the core member 102 so distal interface assembly can rotate about the longitudinal axis of the core member 102. **In** embodiments of the core member 102 that employ both a rotatable stent coupling assembly 120 and a rotatable distal cover 126, the stent 105 can be rotatable with respect to the core member 102 about the longitudinal axis thereof, by virtue of the rotatable connections of the stent coupling assembly 120 and distal cover 126. **In** such embodiments, the stent 105, stent coupling assembly 120 and distal cover 126 can rotate together in this manner about the core member 102. When the stent 105 can rotate about the core member 102, the core member 102 can be advanced more easily through tortuous vessels as the tendency of the vessels to twist the stent 105 and/or core member 102 is negated by the rotation of the stent 105, stent coupling assembly 120, and distal cover 126 about the core member 102. **In** addition, the required push force or delivery force is reduced, as the user's input push force is not diverted into torsion of the stent 105 and/or core member 102. The tendency of a twisted stent 105 and/or core member 102 to untwist suddenly or "whip" upon exiting tortuosity or deployment of the stent 105, and the tendency of a twisted stent to resist expansion upon deployment, are also reduced or eliminated. Further, in some such embodiments of the core member 102, the user can "steer" the core member 102 via the tip coil 108, particularly if the coil 108 is bent at an angle in its unstressed configuration. Such a coil tip can be rotated about a longitudinal axis of the system 100 relative to the stent 105, coupling assembly 120 and/or distal cover 126 by rotating the distal region 106 of the core member 102. Thus, the user can point the coil tip 108 in the desired direction of travel of the core member 102, and upon advancement of the core member the tip will guide the core member in the chosen direction.

With reference to FIGS. 1A and **1B****,** the joining element 200 can include a bumper portion or bumper 210, a proximal portion 212 proximal to the bumper 210, and a nose portion or nose 230 distal of the bumper 210. The elongated shaft 250 can extend through a lumen of the joining element 200 and extend distally to the nose portion 230. As discussed in more detail below, the elongated shaft 250 can mechanically interlock with the proximal portion 212 of the joining element 200, thereby securing the elongated shaft 250 in position relative to the joining element 200. As noted previously, the stent engagement members 123, spacer 125, and distal restraint 121 can be mounted over the elongated shaft 250 at a position distal to the joining element 200. In some embodiments, the stent 105 can be disposed over the stent engagement members 123 with a proximal end of the stent 105 abutting the distal face of the bumper 210. In this configuration, the nose portion 230 can extend partially into a lumen of the stent 105.

The bumper 210 can have an outer diameter that is slightly smaller than the inner diameter of the catheter 101, leaving a radial gap between an outer edge of the bumper 210 and the inner wall of the catheter 101. The nose 230 can have a diameter that is slightly smaller than an inner diameter of a compressed stent 105, allowing for the nose 230 to be positioned within a lumen formed by the stent 105. As previously noted, the joining element 200 can be coupled with the tube 114. The joining element 200 can couple to a distal end portion of the tube 114 so that the bumper 210 is axially disposed between the tube 114 and the stent 105. For example, the distal end portion of the tube 114 can be welded, soldered, and/or otherwise joined to a proximal face 220 of the bumper 210 while the stent 105 is disposed distal to a distal face 222 of the bumper 210. In some embodiments, when the joining element 200 is coupled to the tube 114, the proximal portion 212 of the bumper 210 can be disposed within a lumen formed by the tube 114. In various embodiments, the joining element 200 is configured to abut the proximal end or proximal edge of the stent 105. For example, the bumper 210 can be positioned adjacent the stent 105 so that the bumper 210 abuts the proximal end of the stent 105.

The joining element 200 can couple with the elongated shaft 250. For example, the elongated shaft 250 can interlock with the proximal portion 212 of the joining element 200 and extend through the joining element 200. In some embodiments, the elongated shaft 250 can be coupled with the nose 230 of the joining element 200. For example, the elongated shaft 250 can be welded, soldered, and/or otherwise joined with the nose 230. A portion of the elongated shaft 250 that is distal to the bumper 210 can be configured to receive a medical device such as a stent thereover. For example, the stent coupling assembly 120 can couple to the elongated shaft 250 at a position that is distal to the bumper 210, with the stent coupling assembly 120 configured to engage the stent 105 over this portion of the elongated shaft 250.

As will be described in more detail, the elongated shaft 250 can include a widened region 254 that interlocks with the proximal portion 212 of the joining element 200. By interlocking the elongated shaft 250 with the bumper 210, the joining element 200 can retain the elongated shaft 250 and prevent the elongated shaft 250 from moving distally relative to the core member 102. In some embodiments, the nose 230 can be coupled with the elongated shaft 250 in a manner that prevents the elongated shaft 250 from moving proximally relative to the core member 102. For example, the nose 230 can be welded, soldered, and/or otherwise joined with the elongated shaft 250 so that the elongated shaft 250 cannot be moved proximally relative to the core member 102. Together, the mechanical interlock at the proximal portion 212 of the joining element 200 and the solder joint at the nose 230 of the joining element 200 can prevent any axial movement of the elongated shaft 250 relative to the joining element 200 and/or the tube 114. Additionally, or alternatively, the bumper 210 and the nose 230 can prevent any rotational movement of the elongated shaft 250 relative to the joining element 200 and/or the tube 114. Accordingly, coupling the elongated shaft 250 with the joining element 200 can render the elongated shaft 250 immovable relative to the joining element 200 and/or the tube 114, as the joining element 200 can retain the elongated shaft 250 in place axially and rotationally.

In some embodiments, the joining element 200 can be used to move (e.g., push) the stent 105 distally through the catheter 101. For example, in response to a distal push force applied to the core member 102, the distal face 222 of the bumper 210 can press against the stent 105 and move the stent 105 distally within the catheter 101. When the joining element 200 is configured to push the stent 105 distally, the joining element 200 can be configured to transmit some, most, or all of a distally directed axial (e.g., push) force to the stent 105, wholly or partially in place of the engagement members 123. In such a configuration, the engagement members 123 can be configured to transmit little or no push force to the stent 105 while the stent 105 is delivered distally along the length of the catheter 101. Advantageously, this can reduce or eliminate a tendency of the engagement members 123 to distort the pores of the stent 105 with which the engagement members 123 are engaged when the engagement members 123 are employed to transmit force to and move the stent 105 within the catheter 101. Use of the joining element 200 to move the stent 105 in this manner can also reduce or eliminate axial movement of the stent 105 relative to the core member 102 that sometimes accompanies the pore distortion. In most cases, the vast majority of the travel of the stent 105 within the catheter 101 is in the distal or "push" direction during delivery to the treatment location, in contrast to the relatively short travel involved in resheathing the stent 105, in the proximal or "pull" direction, prior to an eventual final deployment of the stent. Therefore, configuring the joining element 200 to transmit most or all of the push force to the stent 105 can significantly reduce or substantially eliminate such distortion and/or relative axial movement of the stent.

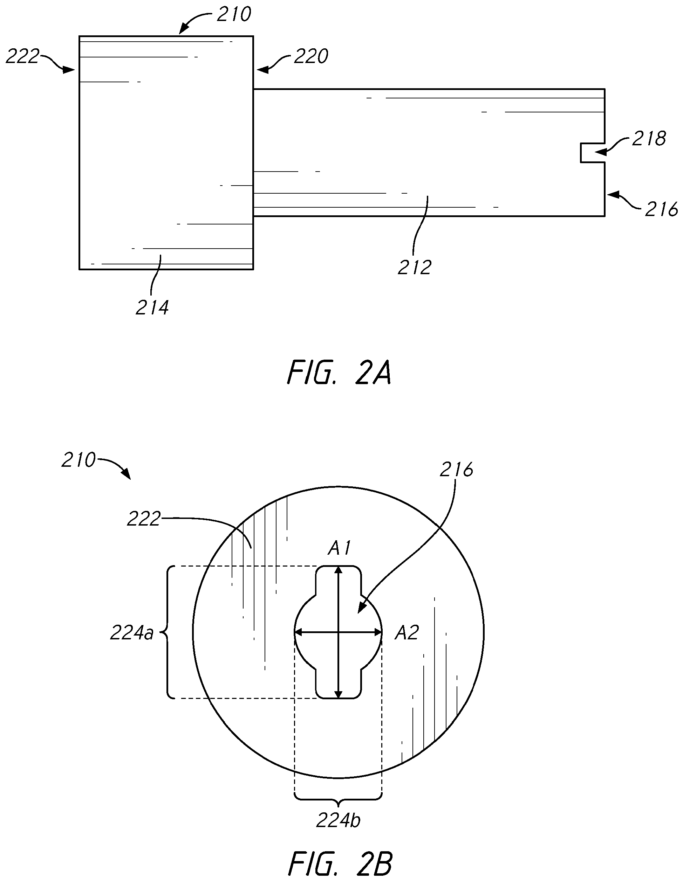

FIG. 2A illustrates a schematic view of the bumper 210 in accordance with one or more embodiments of the present technology. FIG. 2B illustrates an end view of the bumper 210 from FIG. 2A. Referring to FIGS. 2A and 2B together, the bumper 210 can include a generally cylindrical body 214 disposed at the distal end of the proximal portion 212. Although some examples describe the proximal portion 212 as being separate distinct from the bumper 210, in some embodiments the proximal portion 212 and the bumper 210 can be integrally formed and/or combined into the same component. For example, the bumper 210 can define the proximal portion 212 and can include a slot 218 (described below) configured to mechanically interlock with the elongated shaft 250. The proximal portion 212 forms a cylindrical body that extends proximally from the proximal face 220 of the bumper 210. In some embodiments, the width of the proximal portion 212 is smaller than an inner diameter of the tube 114, allowing for the proximal portion 212 to be received within the tube 114. The body 214 of the bumper 210 can have a generally a cylindrical shape that is wider (e.g., having a greater radially outermost dimension) than the proximal portion 212 and can have a width that is slightly smaller than the inner diameter of the catheter 101, leaving a radial gap between an outer edge of the body 214 and the inner wall of the catheter 101.

Additionally, the bumper 210 can define both the proximal face 220 and a distal face 222. An aperture 216 can be formed through the bumper 210 and the proximal portion 212. The aperture 216 can form an irregular shaped lumen throughout the bumper 210 and the proximal portion 212. For example, as illustrated in FIG. 2B, one portion of the aperture 216 can form a rectangular or slot profile while a second portion of the aperture 216 can form a circular or ellipsoid profile. These separate portions of the aperture can define one or more cross-sectional dimensions of different lengths. For example, the slot portion can define a first cross-sectional dimension that has a length 224a and the circular portion can define a second cross-sectional dimension that has a length 224b. In various embodiments, the length 224a can be larger than the length 224b. Additionally, or alternatively, the aperture 216 can define two radial axes or directions. For example, as illustrated in FIG. 2B, the aperture 216 defines a first radial axis A1 along the rectangular profile and a second radial axis A2 along the circular profile. In some embodiments, a slot 218 can be formed at the proximal end of the proximal portion 212. The slot 218 can form a channel within the proximal portion 212 that insects with the aperture 216. In various embodiments, the slot 218 can have a length that is formed at an angle with respect to the axis A1. For example, the slot 218 can be perpendicular to the axis A1. In some embodiments, the slot 218 can have a length that is parallel to the axis A2.

As previously noted, the bumper 210 and the proximal portion 212 can couple with the elongated shaft 250. The aperture 216 and can be sized so that the elongated shaft 250 can be received within the aperture 216. As will be described in more detail, the widened region 254 of the elongated shaft 250 can be positioned within slot 218. Positioning the widened region 254 within the slot 218 interlocks the elongated shaft 250 with the joining element 200 and prevents the elongated shaft 250 from moving distally and/or rotationally relative to the joining element 200.

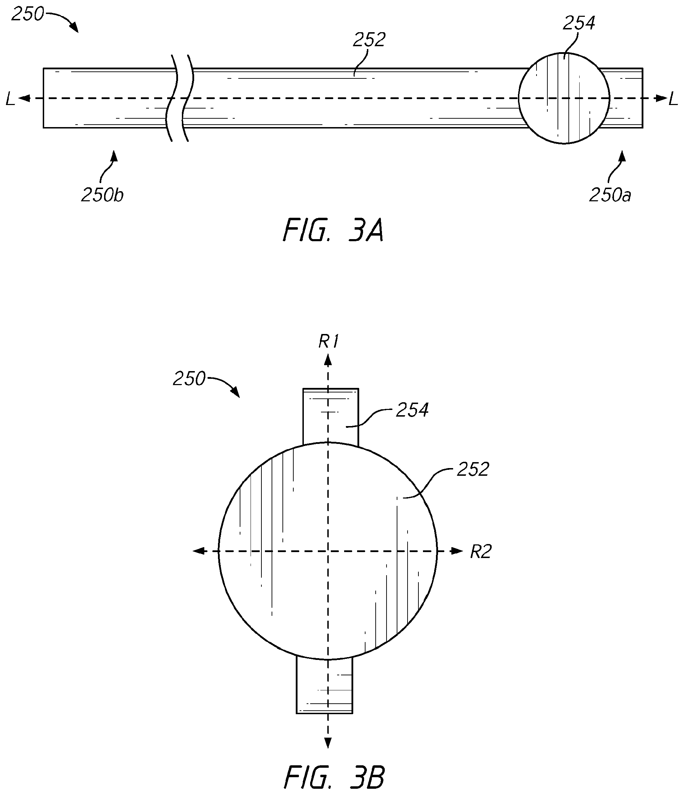

FIG. 3A illustrates a schematic view of the elongated shaft 250 in accordance with one or more embodiments of the present technology. FIG. 3B illustrates an end view of the elongated shaft 250 of FIG. 3A. Referring to FIGS. 3A and 3B together, the elongated shaft 250 can include a body 252 and a widened region 254. The body 252 can extend along an axis L and define a proximal portion 250a and a distal portion 250b. At or proximate the proximal portion 250a, the widened region 254 can be formed in the body 252.

The widened region 254 can be a flattened or "coined" region of the body 252 that forms a cylindrical profile which is normal to a cylindrical profile formed by the body 252. For example, as illustrated in FIGS. 3A and 3B, body 252 forms a cylindrical profile having a height along the axis L while the widened region 254 forms a cylindrical profile having a height along a radial direction R2, with the radial direction R2 being normal to the axis L. This profile of the widened region 254 results in the widened region 254 having cross-sectional dimension that is greater than a cross-sectional dimension of the body 252 along one direction but less than a cross-sectional dimension the body 252 along a separate direction. For example, as illustrated in FIG. 3B, the greatest cross-section dimension of the widened region 254 is greater than greatest cross-section dimension of the body 252 along a radial direction R1 but smaller than greatest cross-section dimension of the body 252 along the radial direction R2. In some embodiments, the widened region 254 can be radially wider than the body 252 along one direction but having substantially the same radial dimension as the body 252 along another direction. In some embodiments, the widened region 254 can include one or more projections (e.g., ridges, bumps, projections, etc.) that project radially outward from the surface of the body 252 at the widened region 254.

The elongated shaft 250 can be sized so that the elongated shaft 250 can be received within the aperture 216. For example, the body 252 of the elongated shaft 250 can have a diameter that is smaller than the length 224b of the aperture 216 while the widened region 254 can have a diameter that is smaller than the length 224a of the aperture 216. By being sized in this manner, both the body 252 and the widened region 254 of the elongated shaft 250 can be received within the aperture 216 of the bumper 210 or proximal portion 212. In some embodiments, the elongated shaft 250 can be received within the aperture 216 when the elongated shaft 250 is in a specific orientation. For example, the widened region 254 can have a diameter that is greater than the length 224b, preventing the widened region 254 from moving through the bumper 210 or proximal portion 212 when the radial direction R1 aligns with the axis A2, but allowing the widened region 254 to move through the bumper 210 or proximal portion 212 when the radial direction R1 aligns with the axis A1. Additionally or alternatively, the widened region 254 can be positioned within the slot 218. The slot 218 can be sized to form a friction fit with widened region 254. When the widened region 254 is positioned within the slot 218, the elongated shaft 250 is prevented from moving distally and/or rotating until the widened region 254 is removed from the slot 218.

The widened region 254 of the elongated shaft 250 can be formed by crimping or crushing a portion of the elongated shaft 250. For example, a press can press into a portion of the elongated shaft 250, forming the widened region 254. The widened region 254 can be formed at any suitable location along the length of the elongated shaft 250, including at a proximalmost end of the shaft 250 or at a region spaced apart from the proximalmost end.

In some embodiments, the widened region 254 can take the form of an engagement feature. The engagement feature can define a first radially outermost dimension along the radial direction R2 and a second radially outermost dimension along the second radial direction R1. In some embodiments, the first radially outermost dimension is smaller than the length 224a of the first cross-sectional dimension and the length 224b of the second-cross sectional dimension. In various embodiments, the second radially outermost dimension is smaller than the length 224a of the first cross-sectional dimension but larger than the length 224b of the second cross-sectional dimension. This configuration allows for the widened region 254 to be moved through the aperture 216 when the widened region 254 is aligned with second cross-sectional dimension (e.g., when the radial direction R1 aligns with the axis A1).

In some embodiments, the widened region 254 of the elongated shaft 250 can take the form of an attachment portion and the portion of the elongated shaft 250 extending through the bumper 210 can take the form of a retention region. The attachment portion of the elongated shaft 250 can extend laterally away from an axis L of the elongated shaft 250 to a greater extent than the retention region of the elongated shaft 250. In some embodiments, the attachment portion is configured to fit through the aperture 216 in a first orientation and to collide with the bumper 210 or proximal portion 212 in a second orientation. For example, when the attachment portion is aligned with the larger portion of the aperture 216, the attachment portion can fit within the aperture 216, but when the attachment portion is not aligned with the larger portion of the aperture 216, the attachment portion will collide with the distal face 222 of the bumper 210 or the proximal face of the proximal portion 212.

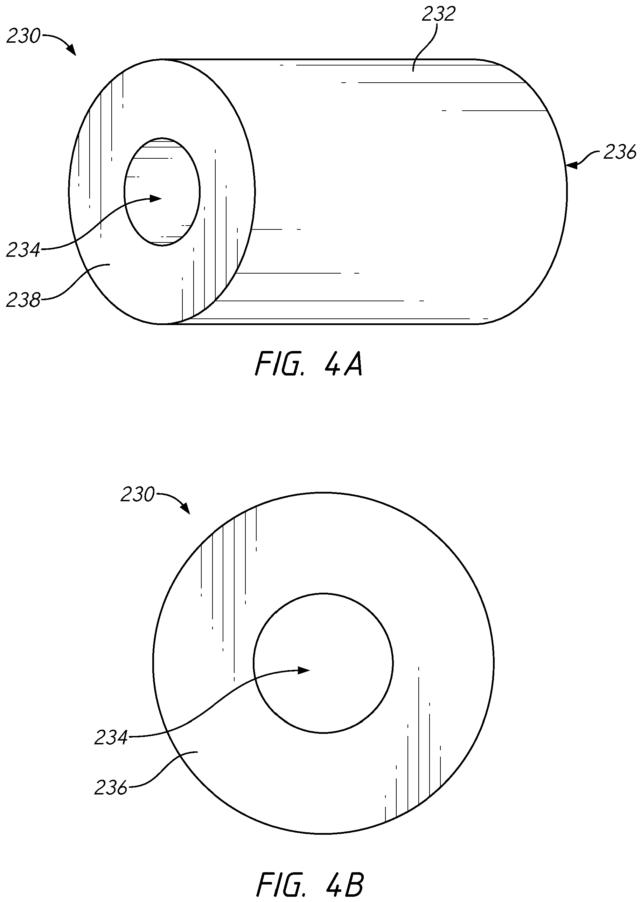

FIG. 4A illustrates an isometric view of the nose 230 in accordance with one or more embodiments of the present technology, and FIG 4B illustrates a view of a proximal face 236 of the nose 230. Referring to FIGS. 4A and 4B together, the nose 230 can have a body 232 with an aperture 234 extending through the body 232. The body 232 can form a cylindrical profile with a proximal face 236 at a first end and a distal face 238 at a second end opposite the first. The diameter or width of the body 232 can be less than the diameter or width of the stent 105, allowing for the nose 230 to be positioned within the lumen of the stent 105 without contacting the stent 105.

As previously noted, the nose 230 can be positioned distal of the bumper 210. In some embodiments, the proximal face 236 of the nose 230 can contact the distal face 222 of the bumper 210. In various embodiments, the nose 230 can be coupled to the bumper 210. For example, the nose 230 can be welded, soldered, or otherwise joined to the bumper 210. Additionally, or alternatively, the nose 230 can be coupled with the elongated shaft 250. At least a portion of the elongated shaft 250 can be disposed within the aperture 234 of the nose 230. While disposed within the aperture 234, the elongated shaft can be welded, soldered, or otherwise joined with the nose 230. In some embodiments, the aperture 216 can align with the aperture 234.

By coupling the nose 230 with the elongated shaft 250, the axial movement of the elongated shaft 250 can be limited or prevented. For example, the elongated shaft 250 can be prevented from moving proximally relative to the core member 102, as the nose 230 would collide with the bumper 210, stopping the elongated shaft 250 from moving proximally. Additionally, or alternatively, the nose 230 can prevent the elongated shaft 250 from rotating relative to the core member 102. For example, the coupling the nose 230 with the bumper 210 and the elongated shaft 250 can stop any rotational movement of the elongated shaft 250 relative to the core member 102. In some embodiments, coupling the nose 230 with the bumper 210 and the elongated shaft 250 can prevent any axial movement of the elongated shaft 250 relative to the core member 102. For example, a fixed connection between the nose 230, bumper 210, and elongated shaft 250 can render the elongated shaft 250 immovable relative to the core member 102.

FIGS. 5A-5E illustrate several schematic views of interlocking the elongated shaft 250 with the joining element 200, in accordance with one or more embodiments of the present technology. FIG. 5A illustrates the elongated shaft 250 being inserted into the aperture 216 of the bumper 210 and FIG. 5A1 shows a cross-sectional view of FIG. 5A along the 5A1 line within FIG. 5A. As illustrated in FIG. 5A, the elongated shaft 250 is inserted into the aperture 216 of the bumper 210 at the distal face 222 of the bumper 210. In some embodiments, the elongated shaft 250 can be inserted into the proximal face of the proximal portion 212. While being inserted into the bumper 210 the widened region 254 is oriented so that the radial direction R1 of the elongated shaft 250 aligns with the axis A1 of the aperture 216. This orientation allows for the widened region 254 to inserted into the aperture 216 without the bumper 210 impeding its movement, as illustrated in FIG. 5A1.

FIG. 5B illustrates the elongated shaft 250 being received in the aperture 216 of the bumper 210 and proximal portion 212 with the widened region 254 being positioned proximal of the proximal portion 212. The elongated shaft 250 can be proximally pushed through the aperture 216 until the widened region 254 is positioned proximally of the proximal portion 212. In this position, the widened region 254 can be spaced apart from the bumper 210, allowing for the elongated shaft 250 to be rotated freely and relative to the bumper 210.

FIG. 5C illustrates the elongated shaft 250 being reoriented after the widened region 254 is positioned proximal of the proximal portion 212. After the widened region 254 is pushed through the aperture 216, and the elongated shaft 250 is free to rotate, the elongated shaft 250 can be aligned with the slot 218. As previously noted, the slot 218 can be formed at an angle with respect the axis A1. Accordingly, to align the widened region 254 with the slot 218, the widened region is rotated so that the radial direction R1 aligns with the length of slot 218. Aligning the widened region 254 with the slot 218 prevents the elongated shaft 250 from being axially moved through the joining element 200, as the widened region 254 will collide with proximal portion 212 and impede any movement through the aperture 216.

FIG. 5D illustrates the elongated shaft 250 interlocked with the proximal portion 212 and FIG. 5D1 shows a cross-sectional view of FIG. 5D along the 5D1 line within FIG. 5D. After the elongated shaft 250 is reoriented so that the widened region 254 is aligned with the slot 218, the elongated shaft 250 can be moved distally so the widened region 254 is received within the slot 218, as illustrated in FIGS. 5D and 5D1. With the widened region 254 being received within the slot 218, the elongated shaft 250 is prevented from moving further distally relative to the proximal portion 212, as the widened region 254 of the elongated shaft 250 is unable to move through the aperture 216 while positioned within the slot 218. Additionally, or alternatively, when the widened region 254 is received within the slot 218, the elongated shaft 250 is prevented from rotating relative to the joining element 200, as the fit between the widened region 254 and slot 218 prevents any substantial rotational movement of the elongated shaft 250 independent from the bumper 210. In some embodiments, the elongated shaft 250 can later be adjusted axially and/or rotationally after the elongated shaft 250 is moved proximally out of the slot 218.

FIG. 5E illustrates the nose 230 being coupled with the elongated shaft 250 after the elongated shaft 250 is interlocked with the bumper 210. The nose 230 can be slid over the elongated shaft 250 until the proximal portion of the nose 230 contacts the distal face 222 of the bumper 210. Once the nose 230 contacts the bumper 210, the nose 230 can be coupled to the elongated shaft 250 by welding, soldering, or otherwise joining the nose 230 and the elongated shaft 250 together. By coupling the nose 230 with the elongated shaft 250, the elongated shaft 250 is prevented from moving proximally relative to the joining element 200, as the contact between the nose 230 and the bumper 210 would impede the elongated shaft 250 and prevent the elongated shaft 250 from moving proximally independent of the joining element 200. In some embodiments, the nose 230 can be coupled to the bumper 210 by welding, soldering, and/or otherwise joining the nose 230 with the bumper 210. By coupling the nose 230 to the bumper 210 and the elongated shaft 250, the elongated shaft 250 can be prevented from rotating independently of the joining element 200.

A method of coupling the elongated shaft 250 with the joining element 200 within a core member 102 will now be described. First, the bumper 210 is coupled to core member 102. The bumper 210 is coupled to the core member 102 by welding, soldering, and/or otherwise joining the bumper 210 to the tube 114. For example, the distal end portion of the tube 114 can be welded to the proximal face 220 of the bumper 210 and/or to the proximal portion 212, coupling the bumper 210 with the tube 114. After the bumper 210 is welded to the tube 114, the elongated shaft 250 is inserted into the aperture 216 of the bumper 210 through the distal face 222 of the bumper 210. In some embodiments, inserting the elongated shaft 250 into the aperture 216 can include aligning the radial direction R1 of the elongated shaft 250 with the A1 axis of the aperture 216. With the elongated shaft 250 positioned within the aperture 216, the elongated shaft 250 is then distally moved through the aperture 216 until the widened region 254 is through aperture 216, proximal to the proximal end of the proximal portion 212 and is free to rotate. Next, the elongated shaft 250 can be rotated so the widened region 254 aligns with the slot 218. Once the widened region 254 aligns with the slot 218, the elongated shaft 250 is moved distally until the widened region 254 is received within the slot 218, interlocking the elongated shaft with the proximal portion 212. Next, the nose 230 is slid over the elongated shaft 250 and positioned adjacent the bumper 210 so that the proximal face of the nose 230 contacts the distal face 222 of the bumper 210. With the nose 230 contacting the bumper 210, the nose 230 is coupled to the elongated shaft 250 and the bumper 210. For example, the nose 230 can be soldered to the elongated shaft 250 and the bumper 210. Coupling the nose 230 with the elongated shaft 250 and bumper 210 along with positioning the widened region 254 within the slot 218 interlocks the elongated shaft 250 with the joining element 200. In some embodiments, the bumper 210 is welded to the tube 114 after the widened region 254 is positioned within the slot 218. In various embodiments, the elongated shaft 250 is initially moved through the proximal portion 212 of the joining element instead of the distal face 222 of the bumper 210.

Operating the system 100 to move the stent 105 will now be described. The stent 105 can be moved distally or proximally within the catheter 101 via the core member 102 and the stent coupling assembly 120. To move the stent 105 out of the catheter 101, the core member 102 is moved distally while the catheter 101 is held stationary, the core member 102 is held stationary while the catheter 101 is withdrawn proximally, or the core member 102 is moved distally while the catheter 101 is simultaneously withdrawn proximally. When the core member 102 is moved distally, the joining element 200 bears against the proximal end or edge of the stent 105 and causes the stent 105 to be advanced distally, and ultimately out of the of the catheter 101. In embodiments in which the engagement members 123 are employed to transmit pushing force to the stent 105, the mechanical engagement or interlock between the engagement members 123 and the stent 105, in response to the application of a distally directed force to the core member 102, causes the stent 105 to move distally through and out of the catheter 101. Conversely, to resheath or otherwise move the stent 105 into the catheter 101, the relative movement between the core member 102 and the catheter 101 is reversed compared to moving the stent 105 out of the catheter 101 such that the proximal region of the restraint 121 bears against the distal region of the spacer 125 and thereby causes the spacer 125, the release members 124, and the engagement members 123 to be retracted into the lumen 111 of the catheter 101. The mechanical engagement between the engagement members 123 and the stent 105 while the engagement members 123 are positioned within the lumen 111 holds the stent 105 with respect to the core member 102 such that proximal movement of the stent 105 relative to the catheter 101 enables re-sheathing of the stent 105 back into the catheter 101. This is useful when the stent 105 has been partially deployed and a portion of the stent 105 remains disposed between at least one of the engagement members 123 (e.g. the first engagement member 123a) and the inner surface of the catheter 101 because the stent 105 can be withdrawn back into the catheter 101 by moving the core member 102 proximally relative to the catheter 101 (and/or moving the catheter 101 distally relative to the core member 102). Resheathing in this manner remains possible until the engagement members 123 and/or catheter 101 have been moved to a point where the first engagement member 123a is beyond the distal opening of the catheter 101 and the stent 105 is released from between the first engagement member 123a and the catheter 101.

### Conclusion

Although many of the embodiments are described with respect to devices, systems, and methods for delivery of stents, tubular implants such as filters, shunts or stent-grafts and other medical devices, other applications and other embodiments in addition to those described herein are within the scope of the present technology, and can be employed in any of the embodiments of systems disclosed herein, in place of a stent as is typically disclosed. Moreover, other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described above with reference to FIGS. 1-5E.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A medical device delivery system comprising:
an elongated tubular member having a distal end portion, a proximal end portion, and a lumen extending therethrough;
a joining element (200) coupled to a distal end portion of the elongated tubular member, the joining element comprising:
a bumper (210) having a distal end portion configured to engage a proximal portion of a medical device;
**characterised in that** the medical device delivery system further comprises
a proximal portion (212) adjacent the distal end portion of the elongated tubular member, wherein the proximal portion defines a slot (218) having a length along a first direction; and an aperture (216) extending through the bumper and the proximal portion, the aperture having a first cross-sectional dimension along a second direction and a second cross-sectional dimension along a third direction different from the second direction, the first cross-sectional dimension being greater than the second cross-sectional dimension, wherein the first direction is about orthogonal to the second direction; and
an elongated shaft (250) having a distal region and a flattened region (254) proximal of the distal region, the flattened region having a greatest cross-sectional dimension that is smaller than the first cross-sectional dimension of the aperture but larger than the second cross-sectional dimension of the aperture, wherein the flattened region is at least partially received within the slot.

2. The medical device delivery system of Claim 1 or 2, wherein, when the flattened region is received within the slot, the flattened region prevents or limits distal movement of the elongated shaft with respect to the joining element.

3. The medical device delivery system of any one of Claims 1 to 3, the joining element further comprising a nose positioned over the elongated shaft and distal of the bumper.

4. The medical device delivery system of Claim 3, wherein the nose is coupled to the elongated shaft.

5. The medical device delivery system of any one of Claims 1 to 4, wherein the elongated shaft is at least one of a wire, hypotube, or coil.

## Patentansprüche

1. Abgabesystem für medizinische Vorrichtungen, umfassend:
ein längliches rohrförmiges Element mit einem distalen Endabschnitt, einem proximalen Endabschnitt und einem sich dort hindurch erstreckenden Lumen;
ein Verbindungselement (200), gekoppelt an einen distalen Endabschnitt des länglichen rohrförmigen Elements, wobei das Verbindungselement umfasst:
einen Anschlag (210) mit einem distalen Endabschnitt, der konfiguriert ist, um mit einem proximalen Abschnitt einer medizinischen Vorrichtung in Eingriff zu kommen;
**dadurch gekennzeichnet, dass** das Abgabesystem für die medizinische Vorrichtung ferner einen proximalen Abschnitt (212) angrenzend an den distalen Endabschnitt des länglichen rohrförmigen Elements umfasst, wobei der proximale Abschnitt einen Schlitz (218) mit einer Länge entlang einer ersten Richtung definiert; und
eine Öffnung (216), die sich durch den Anschlag und den proximalen Abschnitt erstreckt, wobei die Öffnung eine erste Querschnittsabmessung entlang einer zweiten Richtung und eine zweite Querschnittsabmessung entlang einer dritten Richtung, die sich von der zweiten Richtung unterscheidet, aufweist, wobei die erste Querschnittsabmessung größer als die zweite Querschnittsabmessung ist, wobei die erste Richtung etwa orthogonal zu der zweiten Richtung ist; und
einen länglichen Schaft (250) mit einem distalen Bereich und einem abgeflachten Bereich (254) proximal des distalen Bereichs, wobei der abgeflachte Bereich eine größte Querschnittsabmessung aufweist, die kleiner als die erste Querschnittsabmessung der Öffnung, aber größer als die zweite Querschnittsabmessung der Öffnung ist, wobei der abgeflachte Bereich mindestens teilweise in dem Schlitz aufgenommen ist.

2. Abgabesystem für medizinische Vorrichtungen nach Anspruch 1 oder 2, wobei, wenn der abgeflachte Bereich in dem Schlitz aufgenommen ist, der abgeflachte Bereich eine distale Bewegung des länglichen Schafts in Bezug auf das Verbindungselement verhindert oder begrenzt.

3. Abgabesystem für medizinische Vorrichtungen nach einem der Ansprüche 1 bis 3, wobei das Verbindungselement ferner eine Nase umfasst, die über dem länglichen Schaft und distal von dem Anschlag angeordnet ist.

4. Abgabesystem für medizinische Vorrichtungen nach Anspruch 3, wobei die Nase an den länglichen Schaft gekoppelt ist.

5. Abgabesystem für medizinische Vorrichtungen nach einem der Ansprüche 1 bis 4, wobei der längliche Schaft mindestens eines von einem Draht, einem Hypotube oder einer Spule ist.

## Revendications

1. Système de pose de dispositif médical comprenant :
un élément tubulaire allongé ayant une partie d'extrémité distale, une partie d'extrémité proximale et une lumière s'étendant à travers celui-ci ;
un élément de liaison (200) accouplé à une partie d'extrémité distale de l'élément tubulaire allongé, l'élément de liaison comprenant :
une butée (210) ayant une partie d'extrémité distale conçue pour venir en prise avec une partie proximale d'un dispositif médical ;
**caractérisé en ce que** le système de pose de dispositif médical comprend en outre une partie proximale (212) adjacente à la partie d'extrémité distale de l'élément tubulaire allongé, dans lequel la partie proximale définit une fente (218) ayant une longueur le long d'une première direction ; et
une ouverture (216) s'étendant à travers la butée et la partie proximale, l'ouverture ayant une première dimension de section transversale le long d'une deuxième direction et une seconde dimension de section transversale le long d'une troisième direction différente de la deuxième direction, la première dimension de section transversale étant supérieure à la seconde dimension de section transversale, dans lequel la première direction est à peu près orthogonale à la deuxième direction ; et
une tige allongée (250) ayant une région distale et une région aplatie (254) proximale par rapport à la région distale, la région aplatie ayant une plus grande dimension de section transversale qui est inférieure à la première dimension de section transversale de l'ouverture mais supérieure à la seconde dimension de section transversale de l'ouverture, dans lequel la région aplatie est au moins partiellement reçue à l'intérieur de la fente.

2. Système de pose de dispositif médical selon la revendication 1 ou 2, dans lequel, lorsque la région aplatie
est reçue à l'intérieur de la fente, la région aplatie empêche ou limite le déplacement distal de la tige allongée par rapport à l'élément de liaison.

3. Système de pose de dispositif médical selon l'une quelconque des revendications 1 à 3, l'élément de liaison comprenant en outre un nez positionné sur la tige allongée et de manière distale par rapport à la butée.

4. Système de pose de dispositif médical selon la revendication 3, dans lequel le nez est accouplé à la tige allongée.

5. Système de pose de dispositif médical selon l'une quelconque des revendications 1 à 4, dans lequel la tige allongée est au moins l'un parmi un fil, un hypotube ou une bobine.
